# EUROPEAN PATENT SPECIFICATION

(11) **EP 1 046 382 B1**
(45) Date of publication and mention of the grant of the patent: **13.02.2008**
(21) Application number: 00201471.0
(22) Date of filing: 25.04.2000
(51) Int. Cl.: A61F 11/08

(54) **Sound damping filter for ear protector**
Schalldämpfer für Gehörschützer
Filtre amortisseur acoustique pour protecteur d'oreilles

(30) Priority: 23.04.1999 NL 1011877
(43) Date of publication of application: 25.10.2000
(73) Proprietor: Groeneveld Elcea B.V., 5107 RG Dongen (NL)
(72) Inventor: Meeussen, Victor Joseph Anton, 2931 LE Krimpen aan de Lek (NL); Collée, Ronald, 3039 LG Rotterdam (NL)
(74) Representative: Prins, Adrianus Willem

(56) References cited:
- EP-A- 0 333 298
- WO-A-97/27370
- WO-A-98/25558
- FR-A- 2 676 642
- GB-A- 2 075 849
- US-A- 3 842 829
- US-A- 4 540 063
- US-A- 5 488 961

## Description

This invention relates to a sound damping filter for an ear protector placeable in the auditory duct of a person, comprising a sound damping canal with a passage extending from a sound inlet opening to a sound outlet opening.

Such a filter is known from practice and is utilized in a housing placeable in the auditory duct of a person in order to damp the intensity of sound to be supplied to the eardrum of the person. By varying the cross section and the length of the sound damping canal, the level of damping can be influenced, and a sound damping filter can be manufactured which is tailored to a particular application.

A disadvantage of the known filter is that the damping action of the damping canal is always stronger for higher sound frequencies than for lower frequencies. Consequently, the known filter has a damping characteristic rising, for instance, from 20 dB at 125 Hz to 40 dB at 8,000 Hz. A person wearing ear protectors in his ears which are tuned to damping machine noise of a frequency of about 50-500 Hz will perceive sound of a higher frequency, for instance the higher speech frequencies between 1 kHz and 3 kHz, only relatively weakly. Not only does this create the impression for the person that he is closed off from the environment, but also dangerous situations may arise in that the person perceives higher frequencies, for instance warning signals, unduly damped.

Also known are filters for ear protectors where the passage of the damping canal is closed off by means of a membrane, such that, during wearing, between the membrane and the eardrum of the person an amount of air is locked. Although an ear protector with such a filter enables a damping level that remains substantially the same over the audiofrequency range, there are disadvantages associated with this type of ear protector as well. In particular, the locked air volume has as a consequence that a wearer, due to conduction of the bones around the ear, has an enhanced perception of swallowing and chewing sounds, due to the so-called occlusion effect. In addition, the closure may give rise to transpiration in the auditory duct.

The object of the invention is to provide a sound damping filter of the type mentioned in the opening paragraph hereof which does not possess the above-mentioned disadvantages. To that end, a sound damping filter according to the invention comprises a sound damping canal, the passage of which is closed off by means of an air permeable, sheetlike membrane, such that transport of air through the filter is possible. What is thus achieved is that a filter can be composed having a flatter damping characteristic, while through transport of air the occurrence of the occlusion effect and transpiration can be avoided.

The damping canal can then be chosen to have so large a diameter and/or so short a length that an increase of the damping of high frequencies is relatively small, whilst the membrane takes care of sufficient damping of low frequencies.

By adjustment of the geometry of the damping canal, the damping of high frequencies can be adjusted, without the damping for the low frequencies thereby being materially influenced. By adjustment of the geometry of the membrane and of the air permeability of the membrane, the damping of low frequencies can be set, without the damping for the high frequencies thereby being materially influenced.

Although the air permeability of the membrane can already be realized by the use of an air-permeable porous material which is provided with micropores, it is to manufacture the membrane of substantially airtight material in accordance with the present invention in which at least one perforation is provided. By increasing the number of perforations and/or the diameter thereof, the damping of low frequencies by the membrane can be reduced. By providing perforations of substantially equal diameter, the low tone damping can simply and reproducibly be stepwise influenced. What is achieved by choosing the diameter of the perforations to be substantially equal to the thickness of the membrane, is that low tone damping in practice can be set in a simple manner. What is achieved by the use of a flexible membrane is that in particular for higher frequencies a lower damping can be realized.

It is noted that patent publication FR 2 676 642 discloses an ear protector having a passage in which a rigid disc is arranged.

It is further noted that when in this context reference is made to low frequencies, these are understood to include in any case frequencies lower than 1,000 Hz, while higher frequencies are understood to comprise at least frequencies higher than 1,000 Hz.

It is additionally noted that in this context a sheetlike membrane is understood to mean at least a membrane with a nonwoven structure.

Further advantageous embodiments of the invention are described in the dependent claims.

The invention will now be further elucidated on the basis of an exemplary embodiment represented in a drawing. In the drawing:
Fig. 1 shows a sectional side elevation of an ear protector with a sound damping filter according to the invention;
Fig. 2 shows a top plan view of the sound damping filter of Fig. 1 taken along the line II-II; and
Fig. 3 shows a graph in which the damping is represented as a function of the frequency.

It is noted that the figures are only schematic representations of preferred embodiments of the invention. In the figures, the same or like parts have been indicated by corresponding reference numerals.

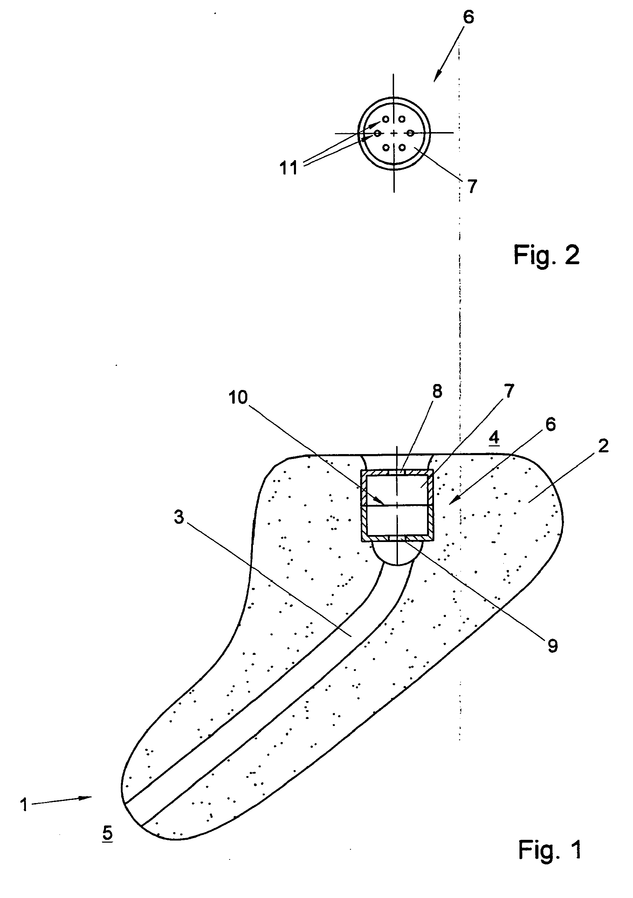

Fig. 1 shows an ear protector 1 having a housing 2 placeable in the auditory duct of a person. Such a housing 2 substantially closing off the auditory duct is generally known and is often designated by those skilled in the art by the name of otoplast. The housing 2 comprises a canal 3 which extends from a side 4 of the housing 2 which in use is located on the ear shell side of the ear, to the side 5 of the auditory duct which in use is located on the eardrum side of the ear. Fitted in the canal 3 is a sound damping filter 6 which is represented in top plan view in Fig. 2. The sound damping filter 6 comprises a sound damping canal 7 with a passage extending from a sound inlet opening 8 to a sound outlet opening 9.

The passage of the sound damping canal 7 is closed off by means of an air-permeable, sheetlike membrane 10. The membrane 10 has a thickness of about 30 µm in a direction transverse to the surface, and is manufactured of substantially airtight, sheetlike, flexible, plastic material. The material is elastic. A suitable material is, for instance, LSR or another silicone material. The thickness of the membrane is preferably less than 0.1 mm and lies advantageously in the range of about 10 µm to about 50 µm. Provided in the membrane 10 are a number of perforations 11 which extend substantially transversely to the membrane surface, preferably perforations of a diameter of about 0.1 mm. For clarification, the perforations are represented on an enlarged scale in Fig. 2.

The membrane 10 can be fitted in the sound damping filter 6 by, for instance, building up the sound damping canal 7 in two parts and glueing the membrane between them. It will be clear to those skilled in the art that there are numerous other possibilities for providing the membrane in the sound damping canal. The membrane 10 can be fitted both tautly and slackly in the damping canal 7.

The extent of damping of sound waves of a low frequency can be influenced by varying the diameter and the number of the perforations 11, with a larger number of perforations and/or a greater diameter of the perforations leading to reduced damping. In addition, the extent of damping can be influenced by adjusting the stiffness of the membrane, with a stiffer membrane leading to increased damping. The damping of sound waves of a high frequency can be influenced, inter alia, by adjusting, inter alia, the length and the diameter of the sound damping canal 7, the thickness of the membrane 10 and optionally the geometry of the canal 3.

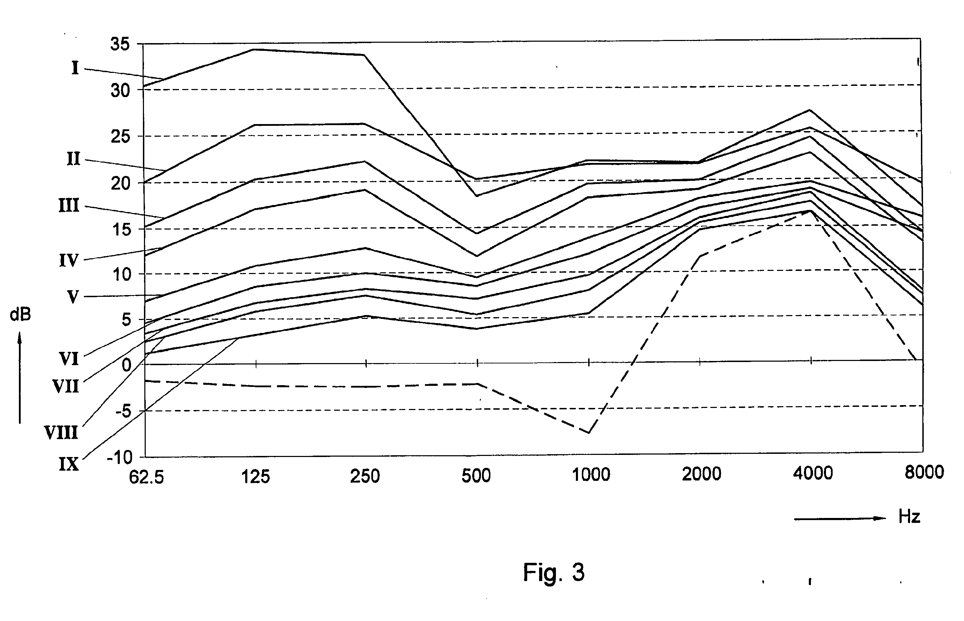

Referring to Fig. 3, the dash line therein represents the damping characteristic of the housing 2 without filter 6 in the canal 3. This damping characteristic depends inter alia on the shape of the auditory duct of the wearer and the selected geometry of the canal 3.

In Fig. 3, the line designated by reference numeral I reflects the damping as a function of the frequency for a filter having a membrane of a diameter of 4 mm and a thickness of 30 µm. By providing a single perforation 11 in the membrane, having a diameter of 0.1 mm, the damping characteristic designated by reference numeral II is achieved. By variation of the number of perforations 11 in the membrane 10, the low-frequency characteristic can be influenced as represented in the figure. The graphs respectively designated by reference numerals III, IV, V, VI, VII, VII, VIII and IX show the behavior for, respectively, four, eight, sixteen, twenty-four, thirty-two, forty and sixty-four perforations 11 of equal diameter.

In this way, starting from the too strong damping characteristic of the closed membrane as indicated by reference numeral I, the damping behavior for low frequencies can be adjusted by providing perforations 11 in the membrane 10. Advantageously, for instance four perforations can be provided, each having a diameter of about 0.1 mm, to obtain in the frequency range up to about 1,000 Hz a substantially flat damping characteristic or a damping characteristic otherwise adjusted to the conditions of use. As appears from Fig. 3, the damping decreases with increasing permeability of the membrane surface. Preferably, therefore, at most 5% of the membrane surface is air-permeable.

By adjustment of the position of the membrane in the sound damping canal 7 or the canal 3, optionally combined with the adjustment of the geometry of the canal 3, it is also possible to realize for the higher part of the frequency range a substantially flat damping characteristic or, if desired, a damping characteristic of a different configuration. Such adjustments to the geometry so as to influence the damping behavior at higher frequencies are known per se.

Advantageously, for instance a canal 3 can be manufactured having a substantially flat damping characteristic for higher frequencies. Thereupon, the damping characteristic for lower frequencies can be adjusted by providing the membrane 10, for instance through laser processing, with perforations 11 until the desired damping characteristic for lower frequencies has been achieved. What can be accomplished by providing perforations 11 of substantially equal diameter is that the adjustment of the damping characteristic for low frequencies can be carried out not only reproducibly, but also relatively simply. It is noted that it is also possible to provide perforations 11 in the membrane 10 prior to placement in the canal 7 of the filter 6 or the canal 3 of the housing 2. To shorten the time required for manufacture, the perforations 11 may optionally be provided simultaneously.

The invention is not limited to the preferred embodiments discussed here, but may also be practiced in other ways, within the scope of the appended claims.

Thus, the membrane may also be fitted directly in the canal 3. In such a case, the sound damping canal 7 and the canal 3 coincide. Also, the sound damping filter may be used in combination with a confection earplug, that is, an ear protector which is not adapted to the auditory duct of a specific person. Further, it is possible that more sound damping canals 7 are present, which may or may not be provided with a membrane 10. Furthermore, the sound inlet opening 8 and/or the sound outlet opening 9 of the sound damping filter 6 can have the same diameter as the sound damping canal 7, and the sound damping canal 7 can include one or more bends and/or have a passage varying in size and/or shape. Also, two or more membranes 10 can be fitted in the damping canal 7, and the membrane may be provided with stiffening ribs or weakening grooves. In addition, the position of the membrane may vary in the longitudinal direction of the damping canal.

It is noted, further, that with increasing diameter of a perforation, damping decreases more strongly than linearly. Although it is possible, in order to achieve a desired damping, to provide one perforation of a relatively large diameter, it may be preferable from the viewpoint of manufacturing technique to provide a larger number of smaller perforations.

It is also noted that the diameter of the perforation(s) is preferably substantially equal to the thickness of the membrane. In particular, the diameter of the perforation(s) is preferably about 0.5 to 2 x the thickness of the membrane, more preferably the diameter of the perforation(s) is 0.8 to 1.2 x the thickness of the membrane.

Such variations will be clear to those skilled in the art and are understood to fall within the framework of the appended claims.

## Claims

1. A sound damping filter (6) for an ear protector (1) placeable in the auditory duct of a person, comprising a sound damping canal (7) with a passage extending from a sound inlet opening (8) to a sound outlet opening (9), the passage of said sound damping canal (7) being closed by means of an air permeable membrane (10), the membrane being manufactured from substantially airlight material in which membrane (10) at least one perforation (11) extending substantially transversely to the membrane surface is provided, such that transport of air through the filter (6) is possible,
**characterized in that** the membrane (10) is elastic and is manufactured from a sheet of flexible material.

2. A sound damping filter (6) according to claim 1, wherein the diameter of the perforation (11) is substantially equal to the thickness of the membrane (10).

3. A sound damping filter (6) according to claim 1 or 2, wherein at least two perforations (11) are provided in the membrane (10), and wherein the diameter of each of the perforations (11) is at least substantially the same.

4. A sound damping filter (6) according to any one of claims 1-3, wherein the membrane (10) is manufactured from elastic material, in particular silicone material.

5. A sound damping filter (6) according to any one of the preceding claims, wherein at most 5% of the membrane surface is air-permeable.

6. A sound damping filter (6) according to any one of the preceding claims, wherein the thickness of the membrane (10) is less than about 0.1 mm.

7. An ear protector (1) comprising a housing (2) placeable in the auditory duct of a person, said housing comprising a sound damping filter (6) according to any one of the preceding claims.

## Patentansprüche

1. Schalldämpfungsfilter (6) für einen im Hörtrakt einer Person platzierbaren Ohrenschutz (1), mit einem Schalldämpfungskanal (7), der einen von einer Schalleinlassöffnung (8) zu einer Schallauslassöffnung (9) verlaufenden Durchlass aufweist, wobei der Durchlass des Schalldämpfungskanals (7) mittels einer luftdurchlässigen Membran (10), die aus einem im Wesentlichen luftdichten Material hergestellt ist, geschlossen ist, wobei in der Membran (10) mindestens eine im Wesentlichen quer zu der Membranoberfläche verlaufende Perforation (11) derart vorgesehen ist, dass der Transport von Luft durch den Filter (6) hindurch möglich ist,
**dadurch gekennzeichnet, dass** die Membran (10) elastisch ist und aus einer Bahn flexiblen Materials hergestellt ist.

2. Schalldämpfungsfilter (6) nach Anspruch 1, bei dem der Durchmesser der Perforation (11) im Wesentlichen gleich der Dicke der Membran (10) ist.

3. Schalldämpfungsfilter (6) nach Anspruch 1 oder 2, bei dem mindestens zwei Perforationen (11) in der Membran (10) vorgesehen sind, und bei der der Durchmesser jeder der Perforationen (11) im Wesentlichen gleich ist.

4. Schalldämpfungsfilter (6) nach einem der Ansprüche 1-3, bei dem die Membran (10) aus einem elastischen Material, insbesondere Siliconmaterial hergestellt ist.

5. Schalldämpfungsfilter (6) nach einem der vorhergehenden Ansprüche, bei dem mindestens 5% der Membranoberfläche luftdurchlässig sind.

6. Schalldämpfungsfilter (6) nach einem der vorhergehenden Ansprüche, bei dem die Dicke der Membran (10) weniger als ungefähr 0,1 mm beträgt.

7. Ohrenschutz (1) mit einem im Hörtrakt einer Person platzierbaren Gehäuse (2), wobei das Gehäuse einen Schalldämpfungsfilter (6) nach einem der vorhergehenden Ansprüche aufweist.

## Revendications

1. Filtre d'amortissement acoustique (6) pour un protecteur d'oreille (1) pouvant être placé dans le conduit auditif d'une personne, comportant un canal d'amortissement acoustique (7) avec un passage s'étendant depuis une ouverture d'entrée acoustique (8) jusqu'à une ouverture de sortie acoustique (9), le passage dudit canal d'amortissement acoustique (7) étant fermé au moyen d'une membrane perméable à l'air (10), la membrane étant fabriquée à partir d'une matière sensiblement étanche à l'air, au moins une perforation (11) s'étendant de manière sensiblement transversale à la surface de membrane étant prévue dans cette membrane (10), de telle sorte qu'un transport d'air à travers le filtre (6) est possible,
**caractérisé en ce que** la membrane (10) est élastique et est fabriquée à partir d'une feuille de matière flexible.

2. Filtre d'amortissement acoustique (6) selon la revendication 1, dans lequel le diamètre de la perforation (11) est sensiblement égal à l'épaisseur de la membrane (10).

3. Filtre d'amortissement acoustique (6) selon la revendication 1 ou 2, dans lequel au moins deux perforations (11) sont prévues dans la membrane (10), et dans lequel le diamètre de chacune des perforations (11) est au moins sensiblement le même.

4. Filtre d'amortissement acoustique (6) selon l'une quelconque des revendications 1 à 3, dans lequel la membrane (10) est fabriquée à partir d'une matière élastique, en particulier une matière à base de silicone.

5. Filtre d'amortissement acoustique (6) selon l'une quelconque des revendications précédentes, dans lequel au moins 5% de la surface de membrane est perméable à l'air.

6. Filtre d'amortissement acoustique (6) selon l'une quelconque des revendications précédentes, dans lequel l'épaisseur de la membrane (10) est inférieure à environ 0,1 mm.

7. Protecteur d'oreille comportant un boîtier (2) pouvant être placé dans le conduit auditif d'une personne, ledit boîtier comportant un filtre d'amortissement acoustique (6) selon l'une quelconque des revendications précédentes.
